Europäisches Patentamt

European Patent Office

Office européen des brevets

(19)

(11) Numéro de publication: **0 202 141 B1**

(12) **FASCICULE DE BREVET EUROPEEN**

(45) Date de publication de fascicule du brevet:
19.06.91

(21) Numéro de dépôt: **86400779.4**

(22) Date de dépôt: **11.04.86**

(51) Int. Cl.⁵: **A61F 2/36**

(54) Tête fémorale pour prothèse de hanche.

(30) Priorité: **12.04.85 FR 8505537**

(43) Date de publication de la demande:
**20.11.86 Bulletin 86/47**

(45) Mention de la délivrance du brevet:
**19.06.91 Bulletin 91/25**

(84) Etats contractants désignés:
**CH DE GB IT LI**

(56) Documents cités:
**EP-A- 0 011 665        EP-A- 0 061 993
EP-A- 0 172 262        DE-A- 2 139 878
DE-B- 2 524 923        FR-A- 1 287 526
FR-A- 2 310 120        FR-A- 2 391 711
FR-A- 2 398 490**

(73) Titulaire: **META CERAM: Société dite
31 rue René Hamon
F-94800 Villejuif (Val de Marne)(FR)**

Titulaire: **LA BIOMECANIQUE INTEGREE: Société dite
Z.I. - 6 rue du Languedoc
F-91220 Bretigny sur Orge (Essonne)(FR)**

(72) Inventeur: **Flegeau, Gérard
26 rue du Fer à Moulin
Paris(FR)**
Inventeur: **Bouvet, Jean-Claude
11 route de Corbeil
Mondeville (Essonne)(FR)**

(74) Mandataire: **Cabinet Pierre HERRBURGER
115, Boulevard Haussmann
F-75008 Paris(FR)**

## Description

La présente invention concerne une tête fémorale pour une prothèse de hanche, selon le préambule de la première revendication.

De manière générale, les prothèses connues se composent suivant le cas d'une partie de prothèse fixée au fémur et qui remplace le col du fémur ainsi que le cas échéant (mais pas dans tous les cas) d'une prothèse de la cavité cotyloïdienne.

La partie de la prothèse fixée au fémur se compose d'une tige solidarisée au fémur préalablement coupé au niveau du col ; cette tige se termine pour certaines prothèses par une extrémité tronconique sur laquelle est emmanchée une tête dite "tête de fémur" en forme de sphère.

Il existe différents types de telles prothèses.

Dans tous les cas, la tige de la prothèse est une pièce métallique ayant des caractéristiques de biocompatibilité, en acier inoxydable, en un alliage chrome-nickel-cobalt ou plus récemment en titane.

La tête fémorale fixée sur l'extrémité conique de la tige a été d'abord fabriquée en un métal du type ci-dessus puis, plus récemment, cette tête a été réalisée en matière céramique.

La tête en matière céramique offre de nombreux avantages et en particulier un meilleur coefficient de frottement dans la cavité cotyloïdienne, une meilleure définition géométrique et une meilleure biocompatibilité.

Toutefois, cette tête en matière céramique présente également des inconvénients dûs à sa fragilité du fait même de l'emmanchement conique qui a tendance sous certains efforts à la faire éclater.

De plus, au moment de l'intervention chirurgicale, le chirurgien dispose en général de trois têtes fémorales différentes qu'il peut fixer sur la partie tronconique de la tige de la prothèse.

Ces têtes fémorales se distinguent par la profondeur de leur cavité tronconique, profondeur variable qui permet d'adapter la géométrie de l'articulation en fonction des nécessités du cas particulier.

Dans le cas d'une tête fémorale en métal, le degré d'engagement de la tête fémorale sur l'extrémité tronconique ou encore la profondeur de la cavité réalisée dans la tête fémorale ne présentent aucun risque pour le patient.

Par contre, dans le cas des têtes en matière céramique, il y a une réelle difficulté à ce niveau car si la tête est soumise à une forte poussée dans la direction axiale, la partie tronconique de la tige travaille par effet de coin et peut faire éclater la tête en matière céramique.

Ce risque existe plus particulièrement dans le cas des têtes en matière céramique ayant une cavité relativement profonde.

Or, comme indiqué ci-dessus, on ne peut exclure le recours à de telles cavités profondes puisque le chirurgien doit disposer de différentes possibilités d'adaptation de longueur, en général trois longueurs.

On connaît déjà une tête fémorale pour prothèse de hanche du type évoqué ci-dessus (document FR - A 2 398 490).

Cette prothèse se compose d'une queue de prothèse destinée à venir dans le canal médullaire du fémur et une tête constituée en céramique ou en un autre matériau dur ; cette tête est fixée de manière amovible sur l'embout conique de la queue de prothèse par l'intermédiaire d'une pièce non précisée. Cette pièce de forme extérieure cylindrique et de forme intérieure conique, de conicité adaptée à celle de la queue de prothèse, présente extérieurement un bourrelet logé dans une rainure périphérique intérieure de la calotte en matière céramique.

Rien n'est dit sur la nature de ce manchon. L'examen des figures montre toutefois que, sous l'effet d'un effort axial, exercé entre la tête de fémur, le bourrelet du manchon crée un effet de coin qui risque de faire éclater la calotte en matière céramique.

De plus, si ce manchon réalisé dans un matériau non précisé, est peu résistant comme il doit l'être pour pouvoir se placer dans la calotte en matière céramique (pour faire passer le bourrelet), la liaison par surface conique entre le manchon et le cône de la queue de prothèse peut induire dans la calotte des efforts provoquant son éclatement.

En résumé, bien que ce document décrive une prothèse formée d'une queue à tête conique et d'une rotule en deux parties, à savoir une partie en matière céramique et une pièce de liaison à surface extérieure cylindrique et surface intérieure conique, le problème de l'absorption des efforts axiaux n'est nullement traité ni même évoqué dans ce document et les moyens ainsi décrits aboutissent de manière quasi certaine à l'éclatement de la rotule.

La présente invention a pour but de remédier à ces inconvénients des techniques connues et se propose de créer une tête fémorale de prothèse de hanche présentant les avantages d'une tête en matière céramique, permettant la combinaison de têtes ou rotules en matière céramique, de dimensions différentes sans présenter de risques d'éclatement de cette tête sous l'effet des efforts axiaux qui peuvent être induits dans cette articulation et sans compliquer la fabrication du produit, de manière prohibitive.

A cet effet, l'invention concerne une tête fémorable pour prothèse de hanche, du type ci-dessus, caractérisée par les moyens décrits dans la partie caractérisante de la première revendication.

Ainsi de façon générale, la tête fémorale pour prothèse de hanche, telle que définie ci-dessus, offre l'avantage très important d'utiliser les caractéristiques de surface et de frottement d'une tête en matière céramique sans avoir la fragilité d'une telle tête notamment si elle est soumise à un effort dans l'axe de l'articulation, par exemple en cas de chute du patient.

La surface d'appui en forme de couronne qui borde la cavité s'appuie contre l'épaulement périphérique du manchon et transmet ainsi à cet épaulement tous les efforts axiaux agissant sur la calotte.

Sur le plan de la fabrication, la tête est d'une réalisation particulièrement simple, puisque la calotte en matière céramique a une géométrie très simple.

Le manchon métallique est lui aussi d'une réalisation très simple puisque le métal peut s'usiner. De plus, pour réaliser une série de têtes fémorales de longueur différente, il suffit de réaliser des manchons différents, les calottes en matière céramique étant les mêmes.

La liaison par engagement entre la calotte en matière céramique et la surface cylindrique du manchon, peut être solidarisée par différents moyens tels qu'une brasure, du ciment ou encore une liaison mécanique telle que par exemple un filetage, un engagement conique ou par clipsage.

Dans tous les cas, que la tête soit très "enfoncée" ou non sur la partie tronconique, le manchon est engagé sur toute la partie tronconique de la tige de la prothèse et assure une excellente tenue.

Dans tous les cas, que la tête soit très "enfoncée" ou non sur la partie tronconique, le manchon est engagé sur toute la partie tronconique de la tige de la prothèse et assure une excellente tenue.

Suivant une autre caractéristique de l'invention, le manchon métallique présente un épaulement périphérique et la calotte sphérique en matière céramique présente une surface d'appui en forme de couronne qui borde la cavité pour venir s'appuyer contre l'épaulement périphérique du manchon et transmettre ainsi à cet épaulement tous les efforts axiaux agissant sur la calotte.

Il est également possible de transmettre les efforts axiaux de la calotte sphérique vers le manchon par la venue en appui du manchon contre le fond de la cavité de la calotte sphérique. Dans ce cas, le manchon ne comporte pas d'épaulement périphérique et la calotte sphérique rejoint la surface extérieure du manchon sans former de surface d'appui en forme de couronne.

La présente invention sera décrite de façon plus détaillée à l'aide des dessins annexés dans lesquels :

- la figure 1 est une vue schématique de l'ensemble d'une prothèse de hanche servant à expliquer le problème posé par la présente invention ;
- la figure 2 est une vue en coupe d'une tête fémorale selon l'invention pour un emmanchement de faible profondeur ;
- la figure 3 est une vue en coupe d'une tête fémorale, selon l'invention, montrant dans sa partie droite une tête pour un emmanchement de profondeur moyenne et à gauche, une tête pour un emmanchement de profondeur importante.

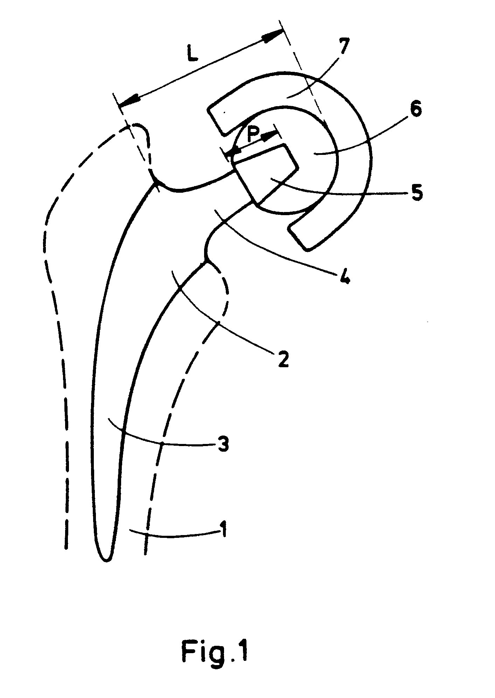

Selon la figure 1, une prothèse de hanche destinée à être fixée sur un fémur 1 se compose d'une tige 2 dont une extrémité 3 est logée dans le fémur après préparation de celui-ci, et dont l'autre extrémité, qui fait partie de la prothèse du col de fémur proprement dite, se compose d'une partie de liaison 4 portant une partie tronconique 5 sur laquelle est emmanchée une tête de fémur 6 selon le principe de l'emmanchement conique ; cette tête 6 vient se loger dans la cavité cotyloïdale 7 naturelle ou de prothèse.

Comme indiqué ci-dessus, la tige 2 est actuellement réalisée en métal et la tête fémorale 6 est réalisée soit en métal soit en matière céramique. Lors d'une intervention chirurgicale, le chirurgien est obligé d'adapter la distance L qui sépare la surface de l'os à l'extrémité de la tête fémorale. Cette distance correspond à un paramètre du patient. Lors de l'intervention le chirurgien met d'abord en place la tige 2 puis adapte sur la partie conique 5 la tête 6 ayant une cavité de profondeur P permettant d'obtenir la distance L qu'il souhaite.

Cette adaptation ne peut se faire qu'après essai sur le patient.

En général, le chirurgien dispose comme déjà indiqué, de trois têtes 6 ayant des cavités de profondeur P différente pour permettre cette adaptation.

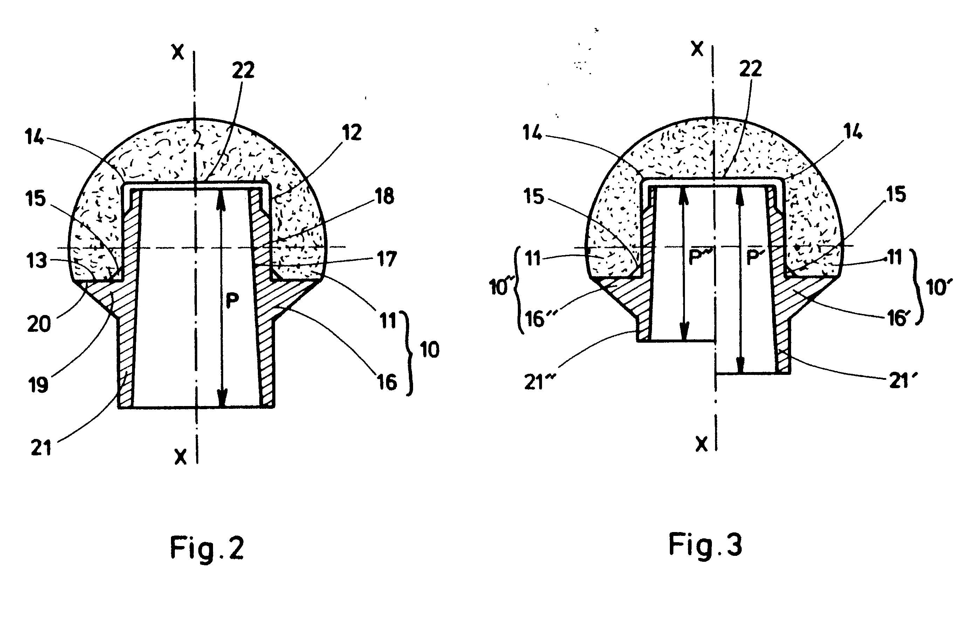

La présente invention sera décrite de façon plus détaillée à l'aide des trois exemples de réalisation représentés aux figures 2 et 3.

Selon la figure 2, la tête fémorale 10 pour une prothèse de hanche se compose d'une partie en forme de calotte sphérique 11 en matière céramique. Cette calotte 11 présente un logement cylindrique 12 et la face avant de la calotte est bordée par une surface 13 en forme de couronne plane. De plus, la calotte 11 présente des congés 14 et des parties coupées 15 pour éviter les amorces de rupture ou pour faciliter l'engagement comme cela sera décrit ci-après.

La tête 10 se compose également d'un manchon 16 métallique. La surface intérieure 17 de ce manchon est conique et sa conicité correspond à la conicité de la surface tronconique de la tige 5 (figure 1) pour permettre un emboîtement conique.

Une partie 18 de la surface extérieure, de ce manchon est de forme cylindrique et son diamètre correspond à celui du logement 12, de façon à permettre l'engagement du manchon 15 dans le logement 12 de la calotte 11.

Enfin, ce manchon 16 présente une collerette périphérique 19 dont la surface 20 tournée vers la calotte 11 forme une surface périphérique d'appui.

Le manchon 16 se poursuit à l'extérieur de la calotte 11 par une jupe 21 plus ou moins longue suivant que la tête fémorale ainsi réalisée doit être plus ou moins emmanchée sur la partie tronconique 5 de la tige.

En d'autres termes, plus la jupe 21 du manchon 16 est longue et plus grande sera la distance L (figure 1). Quelle que soit la distance L à obtenir, la tête fémorale comporte la même calotte sphérique et seule la longueur du manchon métallique est différente de façon que dans tous les cas, d'une part l'enfoncement du manchon dans la calotte sphérique soit le même et que d'autre part le manchon recouvre toute la partie tronconique 5.

Le mode de réalisation de la tête fémorale ci-dessus prévoit l'appui axial entre la calotte et le manchon par la collerette périphérique 19 en évitant tout appui au fond du logement 12.

Il est également possible, selon un mode de réalisation non représenté, de faire l'appui entre le manchon métallique 16 et le fond du logement 12 de la calotte 11. Dans ce cas, il est préférable de supprimer la collerette 19 et de continuer la calotte sphérique 11 jusque sur la surface extérieure du manchon 16 ; ce la revient à réaliser simplement le logement 12 sans réaliser la surface 13 en forme de couronne plane.

Selon un mode de réalisation préférentiel, la solidarisation entre la calotte 11 et le manchon 16 se fait par une brasure. Toutefois d'autres moyens de liaison chimiques ou mécaniques peuvent être utilisés.

A titre d'exemple, la liaison peut être assurée par un adhésif ou un ciment. Elle peut également être faite de façon mécanique par un emboîtement conique, un clipsage etc..

L'emmanchement de la tête fémorale 10 sur la tige, se fait à la manière d'une tête classique en métal et tous les efforts (l'effet de coin) résultant d'une action dans la direction de l'axe X-X, exercés par la partie tronconique 5 sur le manchon 16, sont absorbés par ce manchon 16.

Les deux modes de réalisation de têtes fémorales représentées dans chaque demi-vue de la figure 3, sont semblables au mode de réalisation de la figure 2 et les éléments identiques, les mêmes références, les éléments analogues étant repérés par les mêmes références munies de 'ou'.

Ces deux modes de réalisation diffèrent de celui de la figure 2 uniquement par la longueur P' et P" du manchon conique 16', 16".

Dans la partie droite, le manchon 16' a une longueur moyenne et en partie gauche, la tête fémorale 10" comporte un manchon de profondeur P" qui correspond à la tête fémorale très enfoncée sur la tige.

## Revendications

1. Tête fémorale pour prothèse de hanche, destinée à être fixée sur une tige de prothèse (2) elle-même fixée au fémur (1), cette tige comportant une extrémité tronconique (5) pour recevoir la tête fémorale (6), par emboîtement, tête fémorale formée d'une calotte (11) sphérique en matière céramique ayant un logement (12) recevant un manchon métallique (16) dont la surface intérieure est destinée à être emmanchée sur l'extrémité tronconique (5) de la tige, tête caractérisée en ce que :
   - le manchon (16) a une surface extérieure (18) cylindrique et le logement (12) une surface cylindrique,
   - le manchon métallique et la calotte sphérique (11) sont en appui l'un contre l'autre par des surfaces d'appui constituées sur le manchon métallique par un épaulement périphérique (19) et sur la calotte sphérique (11) en matière céramique par une surface d'appui (13) en forme de couronne qui borde le logement (12), pour venir s'appuyer contre l'épaulement périphérique du manchon,
   - des moyens de liaison reliant le manchon (16) à la calotte sphérique (11).

2. Tête fémorale selon la revendication 1, caractérisée en ce que les moyens de liaison reliant le manchon (16) et la calotte sphérique (11) sont constitués par une brasure.

3. Tête fémorale selon la revendication 1, caractérisée en ce que les moyens de liaison reliant le manchon (16) et la calotte sphérique (11) sont constitués par un liant ou par un moyen mécanique.

4. Tête fémorale selon la revendication 3, caractérisée en ce que le liant est un ciment ou un adhésif et le moyen mécanique est une liaison par filetage, par clipsage ou par emboîtage conique.

## Claims

1. A femoral head for a hip prosthesis intended to be fixed to a prosthesis shaft (2) which is itself fixed to the femur (1), this shaft comprising a frustoconical end (5) for receiving the femoral head (6), by fitting together, said femoral head being formed by a spherical dome (11) of ceramic material having a recess (12) receiving a metal bush (16) whose inner surface is intended to be fitted on the frustoconical end (5) of the shaft, said head being characterised in that :

- the bush (16) has a cylindrical outer surface (18) and the recess (12) has a cylindrical surface ;
- the metal bush and the spherical dome (11) bear against each other with bearing surfaces formed on the metal bush by a peripherical shoulder (19) and on the spherical dome (11) of ceramic material by à ring-shaped bearing surface (13) which borders the recess (12) so as to bear against the peripheral shoulder of the bush,
- connecting means connecting the bush (13) to the spherical dome (11).

2. A femoral head according to claim 1, characterised in that the connecting means connecting the bush (16) and the spherical dome (11) is formed by a brazed joint.

3. A femoral head according to claim 1, characterised in that the connecting means connecting the bush (16) and the spherical dome (11) is formed by a binder or a mechanical means.

4. A femoral head according to claim 3, characterised in that the binder is a cement or an adhesive and the mechanical means is a connection effected by screw thread, clipping or conical interlocking.

**Ansprüche**

1. Femurkopf für eine Hüftgelenkendoprothese, dazu bestimmt, auf einem Schaft einer Prothese (2) befestigt zu sein, welche selbst an einem Oberschenkel befestigt ist, wobei dieser Schaft ein kegelstumpfartiges äußeres Ende (5) aufweist, um durch Zusammenfügen den Femurkopf (6) aufzunehmen, und wobei der Femurkopf eine kugelförmige Kappe (11) aus keramischem Werkstoff ausbildet, mit einem eine metallische Hülse (16) in sich aufnehmenden Lager (12), dessen innere Oberfläche dazu bestimmt ist, auf dem kegelstumpfartigen äußeren Ende (5) des Schaftes festverbunden zu sein, dadurch gekennzeichnet, daß

- die Hülse (16) eine zylindrische äußere Oberfläche und das Lager (12) eine zylindrische Oberfläche hat,

- die metallische Hülse und die kugelförmige Kappe (11) sich gegenseitig abstützen mittels von einem ringförmigen Vorsprung (19) auf der metallischen Hülse gebildete Stützflächen und mittels einer Stützfläche auf der kugelförmigen Kappe (11) aus keramischem Werkstoff in Form eines Kranzes, welcher das Lager (12) umfaßt, so daß er sich gegen den ringförmigen Vorsprung der Hülse abstützt,
- Verbindungsmittel zum Miteinanderverbinden der Hülse (16) mit der kugelförmigen Kappe vorgesehen sind.

2. Femurkopf gemäß Anspruch 1, dadurch gekennzeichnet, daß die die Hülse (16) mit der kugelförmigen Kappe (11) miteinander verbindenden Verbindungsmittel als Lötung ausgebildet sind.

3. Femurkopf gemäß Anspruch 1, dadurch gekennzeichnet, daß die die Hülse (16) und die kugelförmige Kappe miteinander verbindenden Verbindungsmittel als ein Bindemittel oder als ein mechanisches Mittel ausgebildet sind.

4. Femurkopf gemäß Anspruch 3, dadurch gekennzeichnet, daß das Bindemittel ein Zement oder ein Kleber und das mechanische Mittel eine Gewindeverbindung, eine Klipsverbindung oder eine konische Einfügung ist.

**Fig.1**

Fig.2

Fig.3

EP 0 202 141 B1